# EUROPÄISCHE PATENTSCHRIFT

(11) **EP 1 678 118 B1**
(45) Veröffentlichungstag und Bekanntmachung des Hinweises auf die Patenterteilung: **18.04.2012**
(21) Anmeldenummer: 04790484.2
(22) Anmeldetag: 15.10.2004
(51) Int. Cl.: C07C 209/36, B01J 23/89

(54) **VERFAHREN ZUR HERSTELLUNG VON AMINEN**
METHOD FOR THE PRODUCTION OF AMINES
PROCEDE DE FABRICATION D'AMINES

(30) Priorität: 17.10.2003 DE 10349095
(43) Veröffentlichungstag der Anmeldung: 12.07.2006
(73) Patentinhaber: BASF SE, 67056 Ludwigshafen (DE)
(72) Erfinder: VAN LAAR, Frederik, 67117 Limburgerhof (DE); SCHWAB, Ekkehard, 67434 Neustadt (DE); OEHLENSCHLÄGER, Steffen, 67063 Ludwigshafen (DE); VOSS, Hartwig, 67227 Frankenthal (DE); MACKENROTH, Wolfgang, 67089 Bad Dürkheim (DE); MORGENSCHWEIS, Konrad, 01108 Dresden (DE); PENZEL, Ulrich, 01945 Tettau (DE); WEIDNER, Bernd, 01994 Wormlage (DE)
(86) Internationale Anmeldenummer: PCT/EP2004/011642
(87) Internationale Veröffentlichungsnummer: WO 2005/037768

(56) Entgegenhaltungen:
- EP-A- 0 595 124
- EP-A1- 1 518 600
- DE-A- 19 911 865
- YASUYUKI MATSUMURA ET AL.: "Synergic Effect of Nickel and Platinium Supported on Silicia in Catalytic Methanol Decomposition" CHEMICAL COMMUNICATIONS - CHEMCOM., 1997, XP002315664 GBROYAL SOCIETY OF CHEMISTRY.
- A. J. RENOUPREZ ET AL.: "Catalytic Activity of Alumina Supported Platinum-Nickel Alloys" STUDIES IN SURFACE SCIENCE AND CATALYSIS, 1981, Seiten 173-185, XP008042149
- JOSÉ MANUEL DOMINGUEZ ET AL.: "On the Structure and Selectivity of Graphite-Supported Pt-Ni Alloys", JOURNAL OF CATALYSIS, vol. 75 , pages 101-111,

## Beschreibung

Die Erfindung betrifft ein Verfahren zur Herstellung von Aminen durch katalytische Hydrierung der entsprechenden Nitroverbindungen.

Die Herstellung von Aminen, insbesondere von aromatischen Mono-, Di- und/oder Polyaminen durch katalytische Hydrierung der entsprechenden Mono-, Di- und/oder Polynitroverbindungen ist seit langem bekannt und vielfach in der Literatur beschrieben. Ein in der Technik häufig eingesetztes aromatisches Amin ist das Toluylendiamin (TDA), das zu Toluylendiisocyanat weiterverarbeitet werden kann und durch Hydrierung Dinitrotoluol (DNT) hergestellt wird. Ein Problem bei der Hydrierung von DNT ist die verstärkte Bildung von Nebenprodukten, neben Leichtsiedern, zumeist desaminierte und kernhydrierte Produkten, kommen häufig höhermolekulare oder teerartige Produkte vor, die neben der Verringerung der Ausbeute des Verfahrens auch zu einer vorzeitigen Desaktivierung des Katalysators führen können.

Als Hydrierkatalysatoren werden, wie beispielsweise in EP-A-0 124 010 beschrieben, häufig Metalle der VIII. Nebengruppe des Periodensystems, insbesondere Raney-Eisen, Raney-Kobalt und Raney-Nickel, eingesetzt.

Häufig werden für die Hydrierung von Nitroaromaten auch Katalysatoren eingesetzt, die Edelmetalle, insbesondere Palladium, oder auch Platin, enthalten. Bekannt sind hierbei auch Katalysatoren, die Platin und Nickel enthalten.

So beschreibt US 3,127,356 ein Verfahren zur Herstellung von Hydrierkatalysatoren, die für die Hydrierung von DNT zu TDA eingesetzt werden können. Die Katalysatoren enthalten einen Träger, eine oleophile Kohlenstoffkomponente, wie zum Beispiel ein Ruß, auf der die Metalle aufgebracht sind. Dabei liegt das Nickel im Katalysator als Oxid oder Hydroxid vor.

US 5,214,212 beschreibt ein Verfahren zur Kernhydrierung von aromatischen Aminen. Als Katalysator wird ein Edelmetallkatalysator eingesetzt, der zusätzlich mit weiteren Metallen, unter anderem Nickel, dotiert sein kann. Als Edelmetall kann Platin im Gemisch mit anderen Edelmetallen eingesetzt werden. Dabei liegen die Edelmetalle im Katalysator als Metalle und die dotierten Metalle in Form von Salzen vor.

In DE 39 28 329 wird ein Verfahren zur Herstellung von chlorsubstituierten aromatischen Aminen aus den entsprechenden Nitroverbindungen beschrieben. Der bei diesem Verfahren eingesetzte Katalysator besteht aus Aktivkohle als Träger, auf die Platin und ein weiteres Metall, insbesondere Nickel, aufgebracht sind.

In EP 595 124 wird ein Verfahren zur Herstellung von chlorsubstituierten aromatischen Aminen aus den entsprechenden Nitroverbindungen beschrieben. Der verwendete Katalysator enthält Platin und Nickel auf Aktivkohle. Dabei wird zunächst Platin auf der Aktivkohle aufgebracht und reduziert und danach Nickel in Form eines Salzes auf den Träger aufgebracht. Das Nickel liegt bei diesem Katalysator als Hydroxid vor.

In EP 768 917 wird ein Katalysator zur Herstellung von Carbonsäure-Salzen beschrieben. Dieser besteht aus einem Ankermetall, beispielsweise Platin, das teilweise in einem alkaliresistenten Träger eingebettet ist und mindestens teilweise mit einem katalytisch wirkenden nichtedlen Metall, beispielsweise Nickel, durch stromloses Überziehen beschichtet wird. Bei diesem Katalysator liegen die beiden Metalle als getrennte Phasen auf dem Träger vor.

In US 4,185,036 wird ein Verfahren zur Hydrierung von Mischungen von Nitroaromaten beschrieben. Die verwendeten Katalysatoren enthalten Platin und gegebenenfalls ein weiteres Metall, beispielsweise Nickel, auf Aktivkohle. Das weitere Metall liegt dabei in Form des Oxids oder Hydroxids auf dem Träger vor.

DE 199 11 865 und DE 196 36 214 beschreiben Verfahren zur Hydrierung von Dinitrotoluol. Die eingesetzten Katalysatoren enthalten Iridium sowie mindestens ein Dotierungselement, beispielsweise Nickel oder Platin.

WO 03/39743 beschreibt ein Verfahren zur Herstellung von TDA unter Verwendung eines Hydrierkatalysators, bestehend aus Platin, einem weiteren Edelmetall und einem unedlen Metall.

Ständige Aufgabe bei der Hydrierung von DNT zu TDA ist die weitere Erhöhung der Ausbeute und insbesondere die Verbesserung der Selektivität des Verfahrens, um so die Nebenreaktionen, die zur Bildung von hochmolekularen Nebenprodukten oder zur Bildung von Leichtsiedern führen, zurückzudrängen. Weiterhin sollte der Katalysator auch bei höheren Reaktionstemperaturen stabil sein und keine Verschlechterung der Selektivität des Verfahrens zulassen.

Aufgabe der Erfindung war es demzufolge, Katalysatoren für die Hydrierung von aromatischen Nitroverbindungen zu den entsprechenden Aminen, insbesondere von DNT zu TDA, bereitzustellen, die zu einer höheren Ausbeute und Selektivität des Verfahrens führen und auch bei höheren Reaktionstemperaturen keine Verschlechterung der Verfahrensführung zulassen.

Diese Aufgabe konnte überraschenderweise gelöst werden durch die Verwendung von Hydrierkatalysatoren, bei denen Platin und Nickel in Form einer Legierung auf einem Träger vorliegen.

Gegenstand der Erfindung ist somit ein Verfahren zur Herstellung von aromatischen Nitroverbindungen zu den entsprechenden Aminen, insbesondere Toluylendiamin durch katalytische Hydrierung von Dinitrotoluol, in Gegenwart von Hydrierkatalysatoren, dadurch gekennzeichnet, dass Hydrierkatalysatoren eingesetzt werden, bei denen Nickel und Platin auf einem Träger in Form einer Legierung mit einem Atomverhältnis von Nickel zu Platin in der Legierung zwischen 30:70 und 70:30 vorliegen.

Legierungen aus Platin und Nickel mit anderen Atomverhältnissen sind für das erfindungsgemäße Verfahren prinzipiell auch brauchbar, sie führen jedoch, insbesondere bei der Durchführung der Hydrierung bei höheren Temperaturen, zu geringen Ausbeuten an TDA.

Das Atomverhältnis von Nickel zu Platin liegt insbesondere bei zwischen 45:55 und 55:45. Das Atomverhältnis wurde mittels EDXS (Energy Dispersive X-Ray Spectroscopy) bestimmt.

Der Katalysator enthält zumeist ca. 1 bis 15 nm große, feinkristalline Metallpartikeln der Pt-Ni Legierung verteilt auf den Kohlenstoffteilchen. Stellenweise können 1- bis 2-mmgroße Ni-Pt Teilchenagglomerate oder -aggregate, aber auch vereinzelte reine Ni oder Pt Teilchen auf dem Träger vorkommen. Die Elektronenbeugungslinien der Metallteilchen liegen zwischen denen von Pt und Ni, welche die Legierungsbildung zusätzlich belegt. Die Metallpartikeln sind meist polykristallin, und können mit einem hoch-auflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide, wie z.B. ZrO₂, TiO₂, eingesetzt. Bei Graphit sind die HSAG (High Surface Area Graphite) mit einer Oberfläche von 50 bis 300 m²/g besonders bevorzugt. Besonders bevorzugt sind die physisch oder chemisch aktivierte Aktivkohle oder Ruße, wie Acetylenruß.

Der erfindungsgemäß verwendete Katalysator wird vorzugsweise in einer Menge von 0,01 bis 10, vorzugsweise 0,1 bis 5, besonders bevorzugt 0,2 bis 2 Gew.-%, bezogen auf das Reaktionsgemisch, eingesetzt.

Der Katalysator wird zumeist in reduziertem und passiviertem Zustand in den Reaktor eingebracht. Unter dem reduzierten und passivierten Zustand des Katalysators wird verstanden, dass der Katalysator nach der Herstellung aktiviert ist, danach jedoch, aus Sicherheitsgründen, die aktiven Zentren, beispielsweise durch Überleiten von Sauerstoff oder Kohlendioxid, passiviert werden. Alternativ kann der Katalysator unter einer inerten Atmosphäre oder in einem nicht leicht-entzündlichen Lösungsmittel ausgebaut und stabilisiert werden, beispielsweise in Wasser, TDA/Wasser oder höheren Alkoholen, wie Butanol oder Ethylenglykol.

Das erfindungsgemäße Verfahren kann kontinuierlich oder diskontinuierlich unter Verwendung üblicher Reaktoren bei üblichen Verfahrensparametern, wie Druck und Temperatur, durchgeführt werden.

Vorzugsweise wird die erfindungsgemäße Hydrierung bei Drücken im Bereich von 5 bis 100 bar, besonders bevorzugt bei 10 bis 40 bar, insbesondere bei 20 bis 25 bar, durchgeführt.

Bevorzugt wird die erfindungsgemäße Hydrierung bei einer Temperatur im Bereich von 80 bis 250°C, besonders bevorzugt im Bereich von 100 bis 220°C und insbesondere im Bereich 160 bis 200°C durchgeführt.

Zumeist wird die Hydrierung in Form einer kontinuierlichen Suspensionshydrierung in üblichen und geeigneten Reaktoren durchgeführt. Als Reaktoren kommen beispielsweise Rührkessel oder Schlaufenreaktoren, wie zum Beispiel Strahlschlaufenreaktoren, sogenannte Loop-Venturi-Reaktoren, oder Schlaufenreaktoren mit innerer Strömungsumkehr, wie in WO 00/35852 beschrieben, zum Einsatz. Zur Abtrennung der Katalysatoren vom ausgeschleusten Reaktionsgemisch können beispielsweise Querstromfilter eingesetzt werden. Ein derartiges Verfahren ist beispielsweise in WO 03/66571 beschrieben.

Als Hydriergase können beliebige Gase verwendet werden, die freien Wasserstoff enthalten und keine schädlichen Mengen an Katalysatorgiften wie beispielsweise Kohlenmonoxid aufweisen. So können beispielsweise Reformerabgase verwendet werden. Möglich sind auch Gemische aus Wasserstoff mit Stickstoff und/oder Kohlendioxid, wie beispielsweise in DE 10105277 beschrieben. Vorzugsweise wird jedoch reiner Wasserstoff als Hydriergas verwendet.

Die bei der Hydrierung gebildeten Amine werden dem Hydriervorgang kontinuierlich oder diskontinuierlich entzogen und einer Aufarbeitung, beispielsweise einer destillativen Nachbehandlung, unterworfen.

Vorzugsweise werden im Rahmen des erfindungsgemäßen Verfahrens aromatische Nitroverbindungen mit einer oder mehreren Nitrogruppen und 6 bis 18 C-Atomen, bspw. Nitrobenzole, wie z.B. o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2-Dimethyl-4-, 1,4-Dimethyl-2-, 1,3-Dimethyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris-(hydroxymethyl)nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 5 Prozent an vicinalem DNT und 0,5 bis 1,5 % an 2,5- und 3,5-Dinitrotoluol aufweisen, zu den entsprechenden Aminen hydriert.

Im erfindungsgemäßen Verfahren kann die aromatische Nitroverbindung in reiner Form, als Mischung mit dem entsprechenden Di- und/oder Polyamin, als Mischung mit dem entsprechenden Di- und/oder Polyamin und Wasser, als Mischung mit dem entsprechenden Di- und/oder Polyamin, Wasser und einem alkoholischen.Lösungsmittel oder als Mischung mit dem entsprechenden Di- und/oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Sofern ein oben beschriebenes Gemisch eingesetzt wird, liegt das Verhältnis von Aminverbindung zu Wasser vorzugsweise im Bereich von 10:1 bis 1:10, besonders bevorzugt im Bereich von 4:1 bis 1:1 und das Verhältnis des Amin/Wasser-Gemischs zu mindestens einem alkoholischen Lösungsmittel vorzugsweise bei 1000:1 bis 1:1, besonders bevorzugt bei 50:1 bis 5:1.

Wie sich aus dem oben gesagten ergibt, kann beim erfindungsgemäßen Verfahren die Hydrierung unter Abwesenheit oder in Gegenwart eines alkoholischen Lösungsmittels und eines katalysatorreaktivierenden Zusatzes durchgeführt werden.

Sofern ein alkoholisches Lösungsmittel und ein katalysatorreaktivierender Zusatz verwendet werden, können selbstverständlich auch Gemische aus zwei oder mehr davon zugesetzt werden.

Als alkoholische Lösungsmittel werden niedere aliphatische Alkohole mit 1 bis 6 C-Atomen, vorzugsweise Methanol, Ethanol oder Propanol einzeln oder ein Gemisch aus zwei oder mehr davon verwendet.

Als katalysatorreaktivierende Zusätze werden vorzugsweise aprotische Lösungsmittel, insbesondere, DMF, Dioxan oder THF oder ein Gemisch aus zwei oder mehr davon eingesetzt.

Die Menge der eingesetzten alkoholischen Lösungsmittel und der katalysatorreaktivierenden Zusätze ist im Rahmen des erfindungsgemäßen Verfahrens nicht in besonderer Weise beschränkt und kann je nach Bedarf frei gewählt werden.

Überraschenderweise ist es jedoch auch möglich, die Hydrierung aromatischer Nitroverbindungen nach dem erfindungsgemäßen Verfahren ohne die Verwendung von Lösungsmitteln durchzuführen. Bei dieser Verfahrensweise vereinfacht sich die Aufarbeitung des Reaktionsgemisches nach der Hydrierung, außerdem werden Nebenreaktionen mit dem Lösungsmittel völlig unterbunden.

Um Nebenreaktionen zu unterdrücken, ist es bevorzugt, das Verfahren so zu führen, dass der Katalysator an seiner Belastungsgrenze gefahren wird. Dies kann beispielsweise durch die Menge der zudosierten Nitroverbindung, die Menge des Katalysators im Reaktionsgemisch, die Temperatur oder den Druck gesteuert werden.

Unter der Belastungsgrenze des Katalysators wird die Menge der hydrierbaren Stickstoff- und Sauerstoffatome enthaltenden Gruppen verstanden, die vom Katalysator bei gegebenen Druck- und Temperaturbedingungen hydriert werden können. Bei den Stickstoff- und Sauerstoffatome enthaltenden Gruppen kann es sich neben Nitrogruppen auch um Nitrosogruppen und Hydroxylamingruppen handeln.

Die Herstellung der erfindungsgemäßen Katalysatoren erfolgt beispielsweise, indem der Träger vorgelegt und mit einer wässrigen Lösung der Platin- und Nickelsalze zusammengebracht wird. Dabei sollte die Menge des zur Lösung der Salze verwendeten Wassers so bemessen sein, dass eine knetbare Paste entsteht. Vorzugsweise wird das Wasser in einer Menge von 100 bis 200 Gew.-% der Trägermasse eingesetzt. Als Metallsalze kommen insbesondere Nitrate oder Chloride zum Einsatz, wobei Nitrate wegen ihrer geringeren Korrosivität bevorzugt sind. Die Paste wird gemischt und danach das Wasser bei geringem Druck und Temperaturen im Bereich zwischen 50 und 100°C verdampft, beispielsweise in einem Rotationsverdampfer oder einem Ofen. Aus Sicherheitsgründen kann das Verdampfen in einem Stickstoffstrom erfolgen. Die Fixierung der Metalle auf dem Träger bei der Verwendung von Chloriden als Metallsalze kann durch Reduktion mit Wasserstoff erfolgen. Hierbei kann jedoch Korrosion auftreten. Bevorzugt werden die Metalle daher alkalisch fixiert. Dies erfolgt insbesondere durch Zugabe einer wässrigen Lösung von Alkalicarbonaten und nachfolgendes anionfrei waschen des Trägers. Alternativ können die Metalle auch alkalisch, insbesondere bei einem pH-Wert im Bereich von 8 bis 9, aus einer überstehenden Lösung auf den Träger gefällt werden. Danach wird der Träger getrocknet, vorzugsweise wie oben beschrieben, und mit Wasserstoff reduziert. Dies kann beispielsweise in einem Drehkugelofen erfolgen. Vor dem Ausbau des Katalysators wird dieser passiviert, beispielsweise unter einem Inertgas, wie Stickstoff, der Spuren von Luft, vorzugsweise nicht mehr als 10 Vol.-%, enthält.

Durch die Verwendung der erfindungsgemäßen Katalysatoren ist es möglich, die Hydrierung von DNT zu TDA auch bei Temperaturen im Bereich zwischen 160 und 250°C, insbesondere 160 bis 200°C, bei denen sich bei Verwendung herkömmlicher Katalysatoren die Selektivität der Umsetzung stark verschlechtert, durchzuführen. Eine Erhöhung der Reaktionstemperatur ist vorteilhaft da die Löslichkeiten der einzelne Komponente höher ist, als auch nimmt die Reaktionsgeschwindigkeit mit der Temperatur zu. Man kann also die RZA (Raum Zeit Ausbeute) erhöhen, solange die Reaktionsenergie sicher abgeführt werden kann. Eine Erhöhung der Reaktionstemperatur ist vorteilhaft da bei höherer Temperatur die Reaktionsenergie zu nutzen gemacht werden kann durch zum Beispiel Dampferzeugung. Dies ist sicher wirtschaftlich für Temperaturen ab 160° C. Die erzeugte Menge Dampf kann dann eingesetzt werden um z.B. Kühlungsaggregaten zu aktivieren, oder um endotherme Reaktionen zu betreiben.

Die Erfindung soll in den folgenden Beispielen näher erläutert werden.

### Beispiel 1

Ein Aktivkohleträger Norit^{®} SX + wurde in einer Schale vorgelegt und Pt(II)Nitrat für 3 Gew.-% Platin, bezogen auf das Gewicht des Katalysators, und Ni(II)Nitrat-Hexahydrat für 1 Gew.-% Nickel, bezogen auf das Gewicht des Katalysators, wurden in Wasser in einer Menge von 100 Gew.-% der Menge des Trägers gelöst und zu dem Träger gegeben, so dass eine knetbare Paste entstand. Die Paste wurde gut gemischt. Das Lösungsmittel Wasser wurde in einem Rotationsverdampfer unter leichtem Sieden bei 60°C und einem Druck von 0,2 bis 0,4 bar verdampft.Die Metalle wurden auf den Träger durch Zugabe einer Lösung von Natriumcarbonat in einer Menge von 16 Gew.-% der Trägermenge in 100 Gew.-% der Trägermenge Wasser, alkalisch fixiert, und die Probe nitratfrei gewaschen. Der so erhaltene Katalysator wurde bei 80°C getrocknet, bevor er 4 Stunden bei 400°C unter Wasserstoffstrom in einem Drehkugelofen reduziert wurde. Vor dem Ausbau wurde der Katalysator in verdünnter Luft (5 Vol.-% Luft in Stickstoff) bei Raumtemperatur passiviert. Der so erhaltene Katalysator wird als Katalysator A bezeichnet.

Der so erhaltene Katalysator hatte einen Gehalt von 2,9 Gew.-% Platin und 0,97 Gew.-% Nickel. Dies entsprach einem Atomverhältnis von 48:52.

### Beispiel 2 (Vergleich)

Es wurde Verfahren wie in Beispiel 1, jedoch wurde nur Nickelsalz für 0,25 Gew.-% Nickel eingesetzt. Der so erhaltene Katalysator wird als Katalysator B bezeichnet-Das Atomverhältnis von Platin zu Nickel betrug 78:22.

### Beispiel 3 (Vergleich)

Der in Beispiel 1 verwendete Träger wurde in Wasser zu einer 10%igen Suspension suspendiert. Dazu wurden die in Beispiel 1 beschriebenen Metallsalze in dem in Beispiel 1 beschriebenen Verhältnis gegeben und mit Ammoniumformiat unter Rückfluss für 2 Stunden gekocht. Der so erhaltene Katalysator wurde nitratfrei gewaschen. In diesem Procedere wurde das Platin reduziert, das Nickel lag auf den Träger im Form des Hydroxids oder Oxids vor. Der so erhaltene Katalysator wird als Katalysator C bezeichnet.

Platin und Nickel lagen bei diesem Katalysator nicht als Legierung, sondern in Form von diskreten Partikeln vor.

### Beispiel 4

Es wurde verfahren wie in Beispiel 3, es wurde jedoch kein Nickelsalz zugesetzt. Der so erhaltene Katalysator wird als Katalysator D bezeichnet.

Katalysator E (5 % Pd/C)
Kommerzieller, 5 Gew.-% auf Aktivkohle Pd enthaltender Referenzkatalysator
(50 % wasserfeucht).

Katalysator F
Kommerzieller Nickelkatalysator auf einem ZrO₂-Träger.

### Beispiel 5

Hydrierung von DNT zu TDA

Die Hydrierung von DNT zu TDA, wurde in einem 300 ml kontinuierlichen Rührkessel durchgeführt, der Katalysator wurde mechanisch im Reaktor zurückgehalten. Der Katalysator wurde im Wasser suspendiert und im Reaktor gebracht (Katalysatormenge 1 bis 2 Gew.-% des Flüssigkeitsvolumens des Reaktors), auf Temperatur gebracht unter einem H₂-Druck von 22 bar wurde DNT in einer solchen Menge kontinuierlich als Schmelze zudosiert, dass eine Raum-Zeit-Ausbeute von 400 kg_{TDA}/m³,h eingestellt wurde. Proben wurden mittels Gaschromatographie analysiert: Die TDA Ausbeute, Bildung von Hochsiedern und Leichtsiedern wurde verfolgt. Die Raum-Zeit-Ausbeute bei niedriger Temperatur (125°C) lag bei ca. 400 kg-_{TDA}/m³,h, bei höherer Temperatur wurde er variiert 400 bis 700 kg_{TDA}/m³,h. Bei noch höherer Raum-Zeit-Ausbeute stieg der Reaktortemperatur weiter an, bedingt durch die limitierte Kühlleistung des Reaktors.

Die Temperaturen sowie die Ergebnisse sind der Tabelle 1 zu entnehmen.

**Tabelle**

| Beispiel | Katalysator | Temperatur | RZA (kg_{TDA}/m³,h) | TDA (%) |
|---|---|---|---|---|
| 1. Vergleich | F | 125 °C | Ca. 400 | 98,9 |
| | F | 180 °C | Katalysator ist nicht stabil | - |
| 2. Vergleich | E | 140 °C | Ca. 400 | 98,0 |
| | E | 180 °C | Ca. 400 | Ca. 75 |
| 3.Vergleich | D | 125 °C | Ca. 400 | 99,2 |
| | D | 180 °C | Ca. 400 | 98,7 |
| | D | 180 °C | Ca. 700 | 98,8 |
| 4. Vergleich | C | 180 °C | Ca. 700 | 99,1 |
| 5. Vergleich | B | 180 °C | Ca. 700 | 98,9 |
| Erfindungsgemäß | A | 180 °C | Ca. 700 | 99,3 |
| | A | 140 °C | Ca. 400 | 99,5 |

Die Beispiele zeigen, dass der kommerzielle Katalysator F bei niedriger Temperatur sehr gute Ausbeute ergibt, der Katalysator E ist dem Katalysator F deutlich unterlegen. Katalysator D ergibt bei niedriger Temperatur sehr gute Ausbeute, bei höherer Temperatur ist sie gering. Eine Erhöhung der Katalysatorbelastung, beispielsweise durch Erhöhung der Raum-Zeit-Ausbeute kann die TDA Selektivität ein wenig verbessern. Nur mit Katalysator A kann sowohl bei hohen als auch bei niedrigen Temperaturen eine hohe Selektivität erreicht werden.

## Patentansprüche

1. Verfahren zur Hydrierung von aromatischen Nitroverbindungen zu den entsprechenden Aminen in Gegenwart von Hydrierkatalysatoren, **dadurch gekennzeichnet, dass** Hydrierkatalysatoren eingesetzt werden, bei denen Nickel und Platin auf einem Träger in Form einer Legierung mit einem Atomverhältnis von Nickel zu Platin in der Legierung zwischen 30:70 und 70:30 vorliegen.

2. Verfahren nach Anspruch 1, **dadurch gekennzeichnet, dass** als aromatische Nitroverbindung Dinitrotoluol eingesetzt wird.

3. Verfahren nach Anspruch 1, **dadurch gekennzeichnet, dass** das Atomverhältnis von Nickel zu Platin in der Legierung zwischen 40:60 und 60:40 beträgt.

4. Verfahren nach Anspruch 1, **dadurch gekennzeichnet, dass** das Atomverhältnis von Nickel zu Platin in der Legierung zwischen 45:55 und 55:45 beträgt.

5. Verfahren nach Anspruch 1, **dadurch gekennzeichnet, dass** der Katalysator in einer Menge von 0,01 bis 10 Gew.-%, bezogen auf die Reaktionsmischung, eingesetzt wird.

6. Verfahren nach Anspruch 1, **dadurch gekennzeichnet, dass** der Katalysator in einer Menge von 0,1 bis 5 Gew.-%, bezogen auf die Reaktionsmischung, eingesetzt wird.

7. Verfahren nach Anspruch 1, **dadurch gekennzeichnet, dass** der Katalysator in einer Menge von 0,2 bis 2 Gew.-%, bezogen auf die Reaktionsmischung, eingesetzt wird.

8. Verfahren nach Anspruch 1, **dadurch gekennzeichnet, dass** die Hydrierung bei einer Temperatur im Bereich von 80 bis 250°C durchgeführt wird.

9. Verfahren nach Anspruch 1, **dadurch gekennzeichnet, dass** der eingesetzte Katalysator an seiner Belastungsgrenze gefahren wird.

## Claims

1. A process for hydrogenating aromatic nitro compounds to the corresponding amines in the presence of hydrogenation catalysts, which comprises using hydrogenation catalysts in which nickel and platinum are present on a support in the form of an alloy having an atomic ratio of nickel to platinum in the alloy of between 30:70 and 70:30.

2. The process according to claim 1, wherein the aromatic nitro compound used is dinitrotoluene.

3. The process according to claim 1, wherein the atomic ratio of nickel to platinum in the alloy is between 40:60 and 60:40.

4. The process according to claim 1, wherein the atomic ratio of nickel to platinum in the alloy is between 45:55 and 55:45.

5. The process according to claim 1, wherein the catalyst is used in an amount of from 0.01 to 10% by weight, based on the reaction mixture.

6. The process according to claim 1, wherein the catalyst is used in an amount of from 0.1 to 5% by weight, based on the reaction mixture.

7. The process according to claim 1, wherein the catalyst is used in an amount of from 0.2 to 2% by weight, based on the reaction mixture.

8. The process according to claim 1, wherein the hydrogenation is carried out at a temperature in the range from 80 to 250 °C.

9. The process according to claim 1, wherein the catalyst used is used at its loading limit.

## Revendications

1. Procédé pour l'hydrogénation de composés nitrés aromatiques conduisant aux amines correspondantes, en présence de catalyseurs d'hydrogénation, **caractérisé en ce qu'**on utilise des catalyseurs d'hydrogénation dans lesquels du nickel et du platine sont présents sur un support sous forme d'un alliage avec un rapport atomique du nickel au platine dans l'alliage compris entre 30:70 et 70:30.

2. Procédé selon la revendication 1, **caractérisé en ce qu'**on utilise comme composé nitré aromatique le dinitrotoluène.

3. Procédé selon la revendication 1, **caractérisé en ce que** le rapport atomique du nickel au platine dans l'alliage est compris entre 40:60 et 60:40.

4. Procédé selon la revendication 1, **caractérisé en ce que** le rapport atomique du nickel au platine dans l'alliage est compris entre 45:55 et 55:45.

5. Procédé selon la revendication 1, **caractérisé en ce qu'**on utilise le catalyseur en une quantité de 0,01 à 10 % en poids, par rapport au mélange réactionnel.

6. Procédé selon la revendication 1, **caractérisé en ce qu'**on utilise le catalyseur en une quantité de 0,1 à 5 % en poids, par rapport au mélange réactionnel.

7. Procédé selon la revendication 1, **caractérisé en ce qu'**on utilise le catalyseur en une quantité de 0,2 à 2 % en poids, par rapport au mélange réactionnel.

8. Procédé selon la revendication 1, **caractérisé en ce qu'**on effectue l'hydrogénation à une température dans la plage de 80 à 250 °C.

9. Procédé selon la revendication 1, **caractérisé en ce que** le catalyseur utilisé est mis en oeuvre à sa limite de charge.
